# EUROPEAN PATENT APPLICATION

(11) **EP 2 463 658 A1**
(43) Date of publication of application: **13.06.2012**
(21) Application number: 10194775.2
(22) Date of filing: 13.12.2010
(51) Int. Cl.: G01N 33/574

(54) **Biomarkers, uses of biomarkers and a method of identifying biomarkers**

(71) Applicant: Université de Liège, 4031 Angleur (BE)
(72) Inventor: Turtoi, Andrei, 4000 Liège (BE); Castronovo, Vincent, 4000 Liège (BE)
(74) Representative: Atkinson, Jennifer

(57) **Abstract**

The present invention relates to novel breast cancer biomarkers, to an *in vitro* method for identifying accessible breast cancer biomarkers, and to uses of the biomarkers.

## Description

This invention relates to novel biomarkers, to an *in vitro* method for identifying accessible biomarkers for specific diseases, in particular cancer, and to uses of the biomarkers.

A major step in many aspects of research related to diseases such as cancer is the identification of specific and sensitive biomarkers suitable for the development of effective and improved diagnostic, prognostic and therapeutic modalities. An aim of the present invention is to provide novel biomarkers for use as novel diagnostic and/or prognostic markers and/or for use in the development of novel therapeutics.

Whilst mass spectrometry, shot gun proteomics and DNA/RNA microarray analyses have resulted in an increasing list of reported potential tumor biomarkers, very few have found their way into the clinical validation phase and even fewer are used as reliable therapeutic targets or diagnostic markers (Clin.Chem. 48 (2002, whole issue) 1145-1375; Clin. Biochem. 37 (2004, whole issue) 503-647; Mol. Cell. Proteomics 5 (2006, whole issue) S1-S402; J. Proteome Res. 4 (2005, whole issue) 1053-1456).

An aim of the present invention is to provide a method for the identification of potential biomarkers which is targeted to the proteins most likely to be useful. The biomarkers of interest to this invention are accessible biomarkers, that is, biomarkers found in the extracellular matrix or on the outer cell membrane. These proteins are of particular interest because they are reachable by systemically delivered specific agents, such as, monoclonal antibodies which may be radiolabelled or linked to pharmacological compounds.

A major limitation in the identification of valid biomarkers is the scarcity of the tissues from which they need to be recovered. This is particularly true for human pathological tissues, such as cancer lesions, which are available for analysis only in very small amounts, making these samples very precious. Currently available proteomic methods that enable the analysis of accessible proteins from such limited quantity of tissue leave substantial room for improvement. The techniques available attempt to tackle the problem primarily by exploiting the physical location of the biomarkers of interest (Celis, J.E. et al. Mol. Cell. Proteomics 3, 327-344 (2004)) and are to the less extent focused on the chemical propriety. To this end, the use of chemically modified biotin that labels accessible proteins through their free amine groups, combined with streptavidin affinity chromatography has been demonstrated to be a powerful method (Castronovo, V. et al. Proteomics 7, 1188-1196 (2007)). Nevertheless, accessible proteins that do not bear such free amine groups will be omitted from further study. In the method of this invention the fact that most of the outer membrane bound and the extracellular proteins are glycoproteins is exploited.

According to a first aspect the invention provides the use of one or more of myoferlin, CD276, macrophage mannose receptor 2 (CD280), transmembrane 9 superfamily member 3 (EP70-P-iso), EMILIN1, adipocyte enhancer-binding protein 1, agrin, , collagen alpha-1(XII) chain, laminin alpha-5, leucine-rich repeat-containing protein 15, nectin-like protein 2, olfactomedin-like 1, inositol monophosphatase 3 and transforming growth factor beta induced protein ig-h3 (IHC) as a biomarker for breast cancer, or a predisposition thereto. Preferably the invention provides the use of one or more of adipocyte enhancer-binding protein 1, agrin, laminin alpha-5, transforming growth factor beta induced protein ig-h3 (IHC), collagen alpha-1(XII) chain, macrophage mannose receptor 2 (CD280), and nectin-like protein 2 as a biomarker for breast cancer, or a predisposition thereto. Preferably the invention provides the use of one or more of agrin, laminin alpha-5, transforming growth factor beta induced protein ig-h3 (IHC), collagen alpha-1(XII) chain, macrophage mannose receptor 2 (CD280) and nectin-like protein 2 as a biomarker for breast cancer, or a predisposition thereto. Preferably the protein for use as a biomarker is one or more of agrin, laminin alpha-5, myoferlin and olfactomedin-like 1. Preferably the protein for use as a biomarker is adipocyte enhancer-binding protein 1. Preferably the protein for use as a biomarker is agrin. Preferably the protein for use as a biomarker is CD276. Preferably the protein for use as a biomarker is collagen alpha-1(XII) chain. Preferably the protein for use as a biomarker is laminin alpha-5. Preferably the protein for use as a biomarker is leucine-rich repeat-containing protein 15. Preferably the protein for use as a biomarker is macrophage mannose receptor 2 (CD280). Preferably the protein for use as a biomarker is myoferlin. Preferably the protein for use as a biomarker is nectin-like protein 2. Preferably the protein for use as a biomarker is olfactomedin-like 1. Preferably the protein for use as a biomarker is transmembrane 9 superfamily member 3 (EP70-P-iso). Preferably the protein for use as a biomarker is inositol monophosphatase 3. Preferably the protein for use as a biomarker is transforming growth factor beta induced protein ig-h3 (IHC). Preferably the protein for use as a biomarker is EMILIN1.

Preferably the invention provides the use of adipocyte enhancer-binding protein 1 as a therapeutic biomarker for breast cancer.

According to a second aspect the invention provides the use of one or more of one or more of , latent-transforming growth factor beta binding protein 2, asporin adipocyte enhancer-binding protein 1, annexin A6, laminin alpha-2 subunit, laminin subunit alpha-4, mimecan, Ras-related protein Rap-2b, collagen alpha-1(XIV) chain, collagen alpha-3(VI) chain, transforming growth factor beta induced protein ig-h3, latent-transforming growth factor beta binding protein 1, V type proton ATPase catalytic subunit A, protein-glutamine gamma-glutamyl transferase 2, laminin alpha-4 and transmembrane protein 62 as a biomarker for pancreatic cancer, or a predisposition thereto. Preferably the invention provides the use of one or more of one or more of adipocyte enhancer-binding protein 1, annexin A6, laminin subunit alpha-4, collagen alpha-1(XIV) chain, collagen alpha-3(VI) chain, transforming growth factor beta induced protein ig-h3, latent-transforming growth factor beta binding protein 1, latent-transforming growth factor beta binding protein 2, laminin alpha-2 subunit and protein-glutamine gamma-glutamyl transferase 2, laminin alpha-4 and transmembrane protein 62 as a biomarker for pancreatic cancer, or a predisposition thereto. Preferably the protein for use as a biomarker is selected from the group comprising asporin, transforming growth factor beta induced protein ig-h3 and latent-transforming growth factor beta binding protein 2. Preferably the protein for use as a biomarker is adipocyte enhancer-binding protein 1. Preferably the protein for use as a biomarker is annexin A6. Preferably the protein for use as a biomarker is asporin. Preferably the protein for use as a biomarker is laminin alpha-2 subunit. Preferably the protein for use as a biomarker is laminin subunit alpha-4. Preferably the protein for use as a biomarker is mimecan. Preferably the protein for use as a biomarker is Ras-related protein Rap-2b. Preferably the protein for use as a biomarker is collagen alpha-1(XIV) chain. Preferably the protein for use as a biomarker is collagen alpha-3(VI) chain. Preferably the protein for use as a biomarker is transforming growth factor beta induced protein ig-h3. Preferably the protein for use as a biomarker is latent-transforming growth factor beta binding protein 1. Preferably the protein for use as a biomarker is latent-transforming growth factor beta binding protein 2. Preferably the protein for use as a biomarker is V type proton ATPase catalytic subunit A. Preferably the protein for use as a biomarker is protein-glutamine gamma-glutamyl transferase 2. Preferably the protein for use as a biomarker is laminin alpha-4. Preferably the protein for use as a biomarker is transmembrane protein 62.

Preferably the invention provides the use of latent-transforming growth factor beta binding protein 2 as a therapeutic biomarker for pancreatic cancer.

According to a third aspect the invention provides the use of one or more of CD97, prolargin, Thy-1 membrane glycoprotein, transforming growth factor beta induced protein ig-h3 (IHC), a growth hormone inducible transmembrane protein, biglycan, EPCAM, latent-transforming growth factor beta binding protein 2 and EMILIN2 as a biomarker for colorectal carcinoma (CRC) liver metastasis, or a predisposition thereto. Preferably the invention provides the use of one or more of transforming growth factor beta induced protein ig-h3 (IHC) and CD97 as a biomarker for colorectal carcinoma (CRC) liver metastasis, or a predisposition thereto. Preferably the protein for use as a biomarker is one or more of CD97, Thy-1 membrane glycoprotein, transforming growth factor beta induced protein ig-h3 (IHC), a growth hormone inducible transmembrane protein, biglycan, EPCAM and EMILIN2. Preferably the protein for use as a biomarker is CD97. Preferably the protein for use as a biomarker is Thy-1 membrane glycoprotein. Preferably the protein for use as a biomarker is transforming growth factor beta induced protein ig-h3 (IHC). Preferably the protein for use as a biomarker is a growth hormone inducible transmembrane protein. Preferably the protein for use as a biomarker is prolargin. Preferably the protein for use as a biomarker is biglycan. Preferably the protein for use as a biomarker is EPCAM. Preferably the protein for use as a biomarker is latent-transforming growth factor beta binding protein 1. Preferably the protein for use as a biomarker is EMILIN2.

Preferably the invention provides the use of one or more of latent-transforming growth factor beta binding protein 2 and prolargin as a therapeutic biomarker for colorectal carcinoma (CRC) liver metastasis.

Reference to CRC liver metastasis relates to cases where the primary tumour is a colorectal carcinoma which gives rise to a liver metastasis.

According to a fourth aspect the invention provides the use of one or more of adipocyte enhancer binding protein 1 (Uniprot accession no:Q8IUX7), agrin (Uniprot accession no:000468), CD276 (Uniprot accession no:Q5ZPR3), olfactomedin-like 1, myoferlin (Uniprot accession no:Q9NZM1), laminin alpha-5 (Uniprot accession no:O15230), transforming growth factor beta induced protein ig-h3 (Uniprot accession no:Q15582), leucine-rich repeat-containing protein 15 (Uniprot accession no:Q8TF66), transmembrane 9 superfamily member 3 (EP70-P-iso) (Uniprot accession no:Q9HD45), inositol monophosphatase 3 (Uniprot accession no:Q9NX62), EMILIN 1 (Uniprot accession no:Q9Y6C2), collagen alpha-1(XII) chain (Uniprot accession no:Q99715), macrophage mannose receptor 2 (CD280) (Uniprot accession no:Q9UBGO), nectin-like protein 2 (Uniprot accession no:Q9BY67), annexin A6 (Uniprot accession no:P08133), laminin alpha-4 (Uniprot accession no:Q16363), collagen A1 (XIV) (Uniprot accession no:Q05707), collagen A3 (VI) (Uniprot accession no:P12111), latent-transforming growth factor beta binding protein-1 (Uniprot accession no:Q14766), latent-transforming growth factor beta binding protein-2 (Uniprot accession no:Q14767), asporin (Uniprot accession no:Q9BXN1), laminin alpha-2 (Uniprot accession no:P24043), Ras-related protein Rap-2b (Uniprot accession no:Q9Y3L5), V type proton ATPase catalytic subunit A (Uniprot accession no:P38606), protein-glutamine gamma-glutamyl transferase 2 (Uniprot accession no:P21980), transmembrane protein 62 (Uniprot accession no:QOP6H9), mimecan (Uniprot accession no:P20774), prolargin (Uniprot accession no:P51888), biglycan (Uniprot accession no:p2181010), EPCAM (Uniprot accession no:P16422), CD97 (Uniprot accession no:P48960), Thy-1 membrane glycoprotein (Uniprot accession no:P04216) and a growth hormone inducible transmembrane protein as biomarker for cancer, or a predisposition thereto. Preferably the invention provides the use of one or more of olfactomedin-like 1, myoferlin, leucine-rich repeat-containing protein 15, transmembrane 9 superfamily member 3 (EP70-P-iso), inositol monophosphatase 3, EMILIN 1, asporin, Ras-related protein Rap-2b, V type proton ATPase catalytic subunit A, transmembrane protein 62, biglycan, EPCAM, growth hormone inducible transmembrane protein and EMILIN 2 as biomarker for cancer, or a predisposition thereto. Preferably the protein for use as a biomarker is adipocyte enhancer binding protein 1. Preferably the protein for use as a biomarker is agrin. Preferably the protein for use as a biomarker is CD276. Preferably the protein for use as a biomarker is olfactomedin-like 1. Preferably the protein for use as a biomarker is myoferlin. Preferably the protein for use as a biomarker is laminin alpha-5. Preferably the protein for use as a biomarker is transforming growth factor beta induced protein ig-h3. Preferably the protein for use as a biomarker is leucine-rich repeat-containing protein 15. Preferably the protein for use as a biomarker is transmembrane 9 superfamily member 3 (EP70-P-iso). Preferably the protein for use as a biomarker is inositol monophosphatase 3. Preferably the protein for use as a biomarker is EMILIN 1. Preferably the protein for use as a biomarker is EMILIN 2. Preferably the protein for use as a biomarker is collagen alpha-1(XII) chain. Preferably the protein for use as a biomarker is macrophage mannose receptor 2 (CD280). Preferably the protein for use as a biomarker is nectin-like protein 2. Preferably the protein for use as a biomarker is annexin A6. Preferably the protein for use as a biomarker is laminin alpha-4. Preferably the protein for use as a biomarker is collagen A1 (XIV). Preferably the protein for use as a biomarker is collagen A3 (VI). Preferably the protein for use as a biomarker is latent-transforming growth factor beta binding protein-1. Preferably the protein for use as a biomarker is latent-transforming growth factor beta binding protein-2. Preferably the protein for use as a biomarker is asporin. laminin alpha-2. Preferably the protein for use as a biomarker is Ras-related protein Rap-2b. Preferably the protein for use as a biomarker is V type proton ATPase catalytic subunit A. Preferably the protein for use as a biomarker is protein-glutamine gamma-glutamyl transferase 2. Preferably the protein for use as a biomarker is transmembrane protein 62. Preferably the protein for use as a biomarker is mimecan. Preferably the protein for use as a biomarker is prolargin. Preferably the protein for use as a biomarker is biglycan. Preferably the protein for use as a biomarker is EPCAM. Preferably the protein for use as a biomarker is CD97. Preferably the protein for use as a biomarker is Thy-1 membrane glycoprotein. Preferably the protein for use as a biomarker is a growth hormone inducible transmembrane protein.

The proteins and/or peptides referred to in the first, second, third and fourth aspects of the invention are referred to herein as "the biomarker" or "the biomarkers". For example, reference to the biomarkers referred to in the first aspect of the invention is intended to refer to all the proteins recited in the first aspect of the invention.

According to yet another aspect, the invention provides a method for determining the breast cancer status of a subject comprising the steps of:
(a) providing a sample of material from a subject;
(b) determining the level in the sample of one or more of the biomarkers referred to in the first aspect of the invention; and
(c) comparing the level determined in (b) with one or more reference values.

According to yet another aspect, the invention provides a method for determining the pancreatic cancer status of a subject comprising the steps of:
(a) providing a sample of material from a subject;
(b) determining the level in the sample of one or more of the biomarkers referred to in the second aspect of the invention; and
(c) comparing the level determined in (b) with one or more reference values.

According to yet another aspect, the invention provides a method for determining the CRC liver metastasis status of a subject comprising the steps of:
(a) providing a sample of material from a subject;
(b) determining the level in the sample of one or more of the biomarkers referred to in the third aspect of the invention; and
(c) comparing the level determined in (b) with one or more reference values.

According to yet another aspect, the invention provides a method for determining the cancer status of a subject comprising the steps of:
(a) providing a sample of material from a subject;
(b) determining the level in the sample of one or more of the biomarkers referred to in the fourth aspect of the invention; and
(c) comparing the level determined in (b) with one or more reference values.

The samples referred to in step (a) of the preceding aspects of the invention may be obtained from a subject. Preferably the step of obtaining the sample does not form part of the method of the invention.

The method of determining a cancer status or a metastasis status of the invention may be used together with an assessment of clinical symptoms.

The phrase "cancer status" or "metastasis status" includes any distinguishable manifestation of the specific cancer or metastasis to which the biomarkers refer, for example, breast cancer, pancreatic cancer or CRC liver metastasis. For example, cancer status includes, without limitation, the presence or absence of cancer, the risk of developing cancer, the stage of the cancer, the progression of the cancer (e.g. progress of cancer or remission of cancer over time) and the effectiveness or response of a subject to treatment for a cancer.

The method of the invention may be used, for example, for any one or more of the following: to diagnose cancer in a subject; to assess the chance of a subject developing cancer; to advise on the prognosis for a subject with cancer; to monitor disease progression; and to monitor effectiveness or response of a subject to a treatment.

The biomarkers of the invention may be used as one or more of diagnostic biomarkers, prognostic biomarkers and therapeutic biomarkers. In a preferred embodiment, biomarkers which are not explicitly stated to be therapeutic biomarkers are preferably at least one of diagnostic or prognostic biomarkers, or both.

Preferably the method allows the diagnosis of cancer in a subject from the analysis of the level of the one or more biomarkers in a sample provided by/or obtained from the subject.

The method of the invention may also or alternatively be used to develop therapies targeted to the biomarkers. Reference herein to therapeutic biomarkers is intended to refer to biomarkers which can be used as targets for therapy, this may be in addition to or an alternative to the biomarkers being used for diagnostic and/or prognostic purposes. Preferably a therapeutic biomarker represents a protein which can be targeted by a ligand to deliver a drug, for example, the therapeutic biomarker may be recognised by an antibody-drug conjugate wherein the antibody recognises the therapeutic biomarker and delivers the conjugated drug.

The sample material obtained from the subject may comprise whole blood, blood serum, blood plasma, urine, mucous or tissue. Preferably, the sample is a sample of tissues, preferably a sample of tissues suspected to be cancerous, for example a sample of breast tissue, pancreatic tissue or liver tissue, such as a biopsy sample.

Preferably determination of the level of a biomarker in a sample comprises the detection of a polypeptide or an mRNA with at least 65% sequence identity, more preferably at least 70%, 75%, 80%, 85%, 90%, 95% or greater sequence identity, to the published sequence of the biomarker.

Proteins frequently exist in the body, and in samples derived there from, in a plurality of different forms. These forms can result from either or both of pre-and post-translational modification. When detecting or determining the level of a protein in a sample, the ability to differentiate between different forms of a protein depends upon the nature of the difference and the method used to detect or measure the protein level. For example, an immunoassay using a monoclonal antibody will detect all forms of a protein containing the epitope to which the antibody is raised and will not distinguish between different forms. However, a sandwich immunoassay that uses two antibodies directed against different epitopes on a protein will distinguish between forms of the protein that contain both epitopes and those that contain only one of the epitopes.

In a method of the invention the assay method used to determine the level of the biomarker protein preferably detects all forms of the specific biomarker protein. Preferably at least all biologically active forms of the biomarker protein are detected. Preferably, all forms of the biomarker protein with at least 75% or more, preferably at least 80%, 85%, 90% or 95% or more, sequence identity with the published amino acid sequence of the biomarker will be detected in the method of the invention.

Methods of measuring polypeptide/protein/nucleic acid identity are well known in the art. For example the UWGCG Package provides the BESTFIT program which can be used to calculate identity (e.g. used on its default settings) (Devereux et al (1984) Nucleic Acids Research 12, p387-395). The PILEUP and BLAST algorithms can also be used to calculate homology or line up sequences (typically on their default settings), for example as described in Altschul S.F. (1993) J Mol Evol 36:290-300; Altschul, S, F et al (1990) J Mol Biol 215:403-10. Software for performing BLAST analysis is publicly available through the National Centre for Biotechnology Information (http://www.ncbi.nlm.nih.gov/).

The level of the biomarker present in the sample may be determined by any suitable assay which may comprise the use of any of the group comprising enzyme assays, immunoassays, spectrometry, mass spectrometry, Matrix Assisted Laser Desorption/Ionization Time-of-Flight (MALDI-TOF) Mass Spectrometry, microscopy, northern blot, western blot, Southern blot, isoelectric focussing, SDS-PAGE, PCR, quantitative RT-PCR, gel electrophoresis, protein microarray, DNA microarray, and antibody microarray, or combinations thereof.

If antibodies are used one or more antibodies may be synthetic, monoclonal, polyclonal, bispecific, chimeric or humanised. A chimeric antibody includes portions derived from different animals. Humanised antibodies are antibodies from non-human species having one or more complementary determining regions from the non-human species and a framework region from a human immunoglobulin molecule. Chimeric and humanised antibodies can be produced by recombinant techniques well known in the art.

The one or more antibodies may comprise a tag or a label selected from the group comprising a radioactive, a fluorescent, a chemiluminescent, a dye, an enzyme, or a histidine tag or label, or any other suitable label or tag known in the art.

Preferably the reference value, to which the determined levels of the biomarker are compared, is the level of the same protein observed in one or more normal samples. A normal sample preferably refers to a sample of tissue taken from a normal/non diseased location corresponding to the potentially pathologic/diseased tissue being tested. The corresponding normal/non-diseased tissue may be taken from the same or a different individual to the suspected pathologic/diseased tissue. Alternatively, the normal sample may be a sample of normal/non-diseased tissue which is a different tissue to the suspected pathologic/diseased tissue, again this may be taken from the same or a different individual. For example, for a patient with suspected breast cancer, a sample may be taken of the suspected cancer and the normal sample may be taken from non-diseased breast tissue from the same or a different individual.

Alternatively, the reference value may be a previous value obtained for a specific subject. This kind of reference value may be used if the method is to be used to monitor progression of a cancer or to monitor the response of a subject to a particular treatment.

When the determined level of the biomarker is compared with a reference value, an increase or a decrease in the level of the biomarker may be indicative of the cancer status of the subject.

More specifically an increase in the level of the biomarker may be indicative, or diagnostic, of cancer.

Alternatively, a decrease in the level of the biomarker compared to a previously measured level in the same individual may be indicative that a particular therapy has been, or is being, effective.

The method of the invention may also be used to monitor cancer progression and/or to monitor the efficacy of treatments administered to a subject. This may be achieved by analysing samples taken from a subject at various time points following initial diagnosis, and monitoring the changes in the levels of the biomarker, and comparing these levels to normal and/or reference values. In this case reference levels may include the initial levels of the biomarker in the subject, or the level of the biomarker in the subject when they were last tested, or both.

Preferably the method of the invention is carried out *in vitro*.

The subject may be a mammal, and is preferably a human, but may alternatively be a monkey, ape, cat, dog, cow, horse, rabbit or rodent.

According to a yet further aspect the invention provides a method for detecting the presence or absence of a beneficial response in a patient after administration of a therapy, comprising:
(a) providing a biological sample from a patient;
(b) measuring in the sample the level of expression of a biomarker;
(c) comparing levels in (b) to a control value for levels of the biomarker;
(d) determining whether or not the difference in levels between the sample and control reflects a beneficial response in the patient
wherein the biomarker is selected from those identified in the first, second, third or fourth aspect of the invention.

In this aspect of the invention the patient may suffer from one or more of breast cancer, pancreatic cancer, CRC liver metastasis or any other cancer.

This method of the invention may allow the determination of whether or not a particular therapeutic agent is efficacious for the treatment of a particular condition.

Preferably, the control value is the level of a particular biomarker in the patient prior to the administration of a particular therapy, or during the administration period. Preferably, if the level in (b) is lower than the control value this is indicative that the therapy administered is efficacious.

In all aspects of the invention the step of providing a sample preferably does not include the step of obtaining/taking the sample from an individual/patient/subject.

According to another aspect of the invention, there is provided the use of a ligand, preferably a high affinity ligand, directed to a biomarker according to the invention, for the manufacture of a medicament for the therapeutic and/or prophylactic treatment of a human or animal disease. Preferably the medicament is based on the use of specific ligands, preferably antibodies or antibody-drug conjugates.

According to a yet further aspect the invention provides a ligand, preferably a high affinity ligand, directed to a biomarker according to the invention, for use in the therapeutic and/or prophylactic treatment of a human or animal disease. Preferably the ligand is an antibody or antibody-drug conjugate.

According to a still further aspect of the invention, there is provided a method of therapeutic and/or prophylactic treatment of a human or animal disease comprising administering a therapeutically or prophylactically effective amount of a ligand, preferably a high affinity ligand, directed to a biomarker according to the invention. Preferably the ligand is an antibody or an antibody-drug conjugate.

According to another aspect of the invention, there is provided a kit for use in determining the cancer status or metastasis status of a subject, wherein the kit comprises at least one agent for determining the level of one or more biomarkers according to the invention in a sample provided by the subject.

The agent may be an enzyme, an antibody, a nucleic acid, a protein probe, a metabolite or any other suitable composition.

The kit may comprise one or more capture agents for capturing the biomarker, the level of which is to be determined. The capture agent may be one or more antibodies.

The kit may comprise instructions for suitable operational parameters in the form of a label or separate insert. The instructions may inform a consumer about how to collect the sample, and/or how to wash the capture agent.

According to a yet further aspect, the invention provides the use of the determination of the levels of one or more biomarkers according to the invention as a means of assessing the cancer status in an individual.

According to another aspect the invention provides a method of treating cancer in a subject comprising administering to the subject an agent capable of modulating the level of a biomarker according to the invention in the subject.

The agent may act at the transcriptional, translation and/or post translational level.

According to a further aspect the invention provides a method of identifying compounds for treating cancer comprising screening for one or more compounds that modulate the level of one or more biomarkers according to the invention.

According to another aspect of the invention, there is provided an *in vitro* method for the analysis of accessible proteins in a sample comprising:
providing a sample;
labelling accessible proteins in the sample with a labelling reagent;
isolating and recovering proteins labelled with the labelling reagent from the sample;
digesting the remaining, non-labelled, proteins in the sample;
isolating and recovering glycopeptides from the digested sample;
recovering non-glycosylated peptides that were not recovered in the isolation of labelled proteins or glycopeptides;
analysing the recovered proteins/peptides.

Surprisingly the combination of steps in the method of the invention significantly increases the potential to identify accessible proteins from a sample, compared to performing any of the steps alone.

The method of the invention provides an improved method for the identification of accessible biomarkers from pathologic/diseased tissue samples, such a tissue biopsies. The accessible biomarkers/proteins may be membrane bound proteins or proteins secreted into the extracellular space/matrix.

Preferably the accessible proteins can be reached by systemically delivered agents, for example, systemically delivered antibodies. Preferably this is achieved because the accessible proteins are more exposed to the interstitial fluid than other proteins.

The method of the invention preferably allows the number of biomarkers identified in a precious sample, such as a biopsy, to be maximised whilst still only considering accessible proteins.

By analysing the accessible proteins the method allows biomarkers to be identified which can be used for a number of purposes, including one or more of the following, disease diagnosis, disease prognosis, monitoring disease progression, monitoring the efficacy of a treatment, and for targeting potential therapies to cells or tissues expressing the biomarker. Examples of potential therapies include antibodies, antibody fragments, drugs, prodrugs or other ligands which may be targeted to a specific biomarker.

Preferably the method of the invention may be used to screen for/identify biomarkers for specific diseases wherein the biomarkers are accessible from the extracellular space in a tissue or cell sample.

The method of the invention preferably allows a minimal amount of material to be used, making it suitable for the quantitative analysis of scarce biopsy tissue. The method may allow the number of identified valuable biomarkers to be maximized from a precious sample, whilst preserving the quality of these proteins and thus allowing potentially accessible and hence relevant biomarkers to be identified.

The labelling reagent may be any reagent capable of labelling a protein via a characteristic functional group, wherein the labelling reagent then allows the enrichment of labelled proteins. An example of a suitable labelling reagent is a reactive biotin, preferably a biotin reactive ester derivative, which can be used to label primary amines on proteins/peptides in a sample. In the method of the invention the sample is preferably a tissue sample in which the cells are substantially intact and have substantially intact membranes, such that when a labelling reagent is added it cannot penetrate the cells and thus substantially only extracellular or membrane bound proteins are labelled.

If biotin is used as the labelling reagent the high affinity of biotin for avidin may then be exploited to isolate and recover biotin labelled proteins. Other suitable reagents for labelling accessible proteins include a reagent which binds to sugar moieties on proteins or peptides and magnetic beads attached to a functional group that will react with sugar moieties or primary amines.

The method of the invention further includes enriching for accessible proteins by recovering glycosylated peptides. Glycosylated peptides are often membrane associated or secreted, and hence are accessible.

Glycosylated is used herein to refer to proteins or peptides which have an attached saccharide group. The terms glycoprotein/glycopeptide are used interchangeable herein with glycosylated protein/glycosylated peptide, and are intended to have the same meaning.

The sample may be a cell or tissue sample. Preferably the sample is a tissue sample. The tissue sample may be a pathologic tissue sample, that is, from a diseased tissue. Alternatively the tissue sample may be a sample of normal, non diseased, tissue. Where the tissue sample is a pathologic tissue sample the tissue may be selected from tumour tissue, inflamed tissue, atheromatatic tissue, and tissue resulting from a degenerative, metabolic or genetic disease. Preferably the sample is a tissue biopsy.

Reference herein to a normal/non-diseased tissue refers to either normal/non-diseased tissue corresponding to the pathologic/diseased tissue from the same individual or from a different individual. The two tissue samples, normal and pathologic, may be taken from the same location, for example both liver or both breast, or from different locations, in the same or different individuals. For example, the pathologic tissue may be breast tissue and the normal tissue may be a sample of non-diseased breast tissue from the same individual, or a sample of non-diseased breast tissue from a different individual, or a sample of a different tissue from the same or a different individual. Preferably the normal/non-diseased tissue is a sample of the same type of tissue as the pathologic/diseased tissue.

The term "biomarker" is used herein with reference to the method of the invention to refer to any protein or peptide (which may be modified, for example glycosylated) whose level of expression in pathologic tissue allows the tissue to be distinguished from a normal tissue.

Preferably proteins in the sample are labelled with a labelling reagent by immersing the sample in a solution comprising the labelling reagent. Preferably the immersion allows accessible proteins to be labelled with the labelling reagent. The labelling reagent, such as a biotin derivative, is preferably able to covalently link onto accessible primary amines in a protein. Thus only proteins with accessible primary amines will be labelled. Preferably the sample is immersed in a solution of reactive biotin ester derivatives in order to label the accessible proteins.

The sample immersed in the labelling reagent containing solution is preferably a native sample wherein only accessible proteins are labelled, and non-accessible proteins are not, or are essentially not, labelled.

A native sample means that the sample has not been denatured or fixed prior to immersion in the labelling reagent containing solution. Preferably upon immersion of the sample in the labelling reagent containing solution many of the accessible proteins become labelled with the labelling reagent. However, as not all proteins are able to be labelled, further protein/peptide recovery steps are included in the method of the invention to maximise the biomarkers identified.

After the labelling step the sample is preferably subjected to a solubilisation step wherein only the soluble fraction is retained and used in the rest of the method. Preferably the insoluble fraction is discarded.

If the proteins have been labelled with biotin, or a biotin derivative, the labelled proteins may be recovered using streptavidin, preferably using streptavidin beads. Biotinylated proteins/peptides are easily purified due to the highly specific interaction between biotin and streptavidin. This interaction is strong even in the presence of lysis buffers containing strong detergents, thus minimising nonspecific binding during purification. The biotinylated proteins may be eluted from the streptavidin beads using DTT. The eluted protein may then be digested, for example with trypsin, to provide peptide fragments for analysis by mass spectrometry.

Preferably the non-labelled proteins are digested using any proteinase (like trypsin) to produce shorter peptides before the recovery of glycosylated peptides.

Glycoproteins/glycopeptides, preferably glycopeptides, within the remaining proteins/peptide may be isolated by first oxidising sugar moieties on the proteins/peptides. The sugar moieties may be oxidised by using a periodate solution, and then isolating any proteins/peptides with oxidised sugar moieties, for example by using a hydrazide resin. The bound proteins/peptides may be released from the hydrazide using PNGase F. Other methods to isolate glycoproteins/glycopeptides are known and may be used in the method of the invention.

The remaining non-labelled and non-glycosylated proteins/peptides, preferably peptides, are then recovered and are also analysed, for example, by mass spectrometry.

Preferably the remaining non-labelled and non-glycosylated peptides are recovered in the flow through fraction from the method used to recover glycosylated proteins/peptides.

Preferably the recovered proteins/peptides are analysed to determine their sequence and hence the identity of the protein from which they are derived. The recovered proteins/peptides may be analysed by mass spectrometry. Preferably mass spectrometry analysis provides sequence information relating to the proteins/peptides which can then be compared to protein/peptide sequence databases to allow the protein/peptide to be identified.

Preferably if mass spectrometry is used it comprises sequencing by tandem mass spectrometry.

Preferably the analysis of the non-glycosylated peptides recovered allows the identification of glycosylated peptides (recovered in the previous step of the method of the invention) which cannot be confidently assigned to a particular protein based on the analysis of the glycosylated peptide alone. More specifically, the identification of non-glycosylated peptides which are found in a glycosylated protein may allow an isolated glycosylated peptide to be more confidently assigned to a particular glycosylated protein.

Analysis of the non-glycosylated peptides may also allow non-glycosylated, but accessible, proteins to be identified.

Preferably the glycosylated and the non-glycosylated peptides are all analysed by mass spectrometry.

The method of the invention may include the further step of comparing the recovered proteins/peptides from a pathologic tissue sample to the recovered proteins/peptides from a normal tissue sample.

Preferably by comparing the differential expression of recovered proteins/peptides from a pathologic tissue sample with that of a normal sample, potential biomarkers for the disease of the pathologic tissue sample may be identified.

Preferably, by identifying recovered proteins/peptides which have a higher expression in the pathologic tissue sample compared to the normal tissue sample, one or more biomarkers for a specific disease may be identified. The identified biomarkers, due to the nature of the method of the invention, are preferably accessible *in vitro* and *in vivo* from the extracellular space.

The method of the invention may be applied to any tissue and any condition/disease, and because it considers accessible proteins it may, in addition to identifying biomarkers, allow custom therapies to be developed targeted to highly accessible targets. For example, antibody based treatments may be targeted to the biomarker.

In a preferred embodiment of the invention the labelling and recovery steps are performed using a pathologic tissue sample and a normal tissue sample and the differential expression of recovered proteins/peptides are compared. More preferably the method comprises the steps of:
providing a sample of normal tissue and a sample of pathologic tissue;
labelling the accessible proteins in each sample separately with a labelling reagent;
isolating and recovering labelled proteins from each sample;
digesting the remaining, non-labelled, proteins in each sample;
isolating and recovering glycopeptides from each digested sample;
recovering non-glycosylated peptides from each sample that were not recovered in the isolation of labelled proteins or glycopeptides;
analysing all the recovered proteins and peptides;
determining the differential expression pattern of the recovered proteins/peptides in the pathologic sample compared to the normal sample;
identifying proteins/peptides that have a higher expression in the pathologic tissue sample compared to the normal tissue sample, or which are more frequently expressed in the pathologic tissue sample compared to the normal tissue sample. These proteins/peptides may be used as biomarkers for the pathologic tissue, preferably they are accessible for high affinity ligands from the extracellular space.

Preferably the biomarker is expressed in the pathologic tissue and not the normal tissue.

The purified labelled proteins may be cleaved after purification to smaller peptides before further analysis. The cleavage is preferably by proteolytic digestion, this may be achieved using trypsin.

According to a further aspect the invention provides a biomarker identified by the method of the invention.

According to another aspect of the invention, there is provided the use of a ligand, preferably a high affinity ligand, directed to a biomarker identified by the method of invention, for the manufacture of a medicament for the therapeutic and/or prophylactic treatment of a human or animal disease. Preferably the medicament is based on the use of specific ligands, preferably antibodies or antibody-drug conjugates.

According to a yet further aspect the invention provides a ligand, preferably a high affinity ligand, directed to a biomarker identified by the method of invention, for use in the therapeutic and/or prophylactic treatment of a human or animal disease. Preferably the medicament is based on the use of specific ligands, preferably antibodies or antibody-drug conjugates.

According to a still further aspect of the invention, there is provided a method of therapeutic and/or prophylactic treatment of a human or animal disease comprising administering a therapeutically or prophylactically effective amount of a ligand, preferably a high affinity ligand, directed to a biomarker identified by the method of invention. Preferably the ligand is an antibody or an antibody-drug conjugate.

It will be appreciated that optional features applicable to one aspect or embodiment of the invention can be used in any combination, and in any number. Moreover, they can also be used with any of the other aspects or embodiments of the invention in any combination and in any number. This includes, but is not limited to, the dependent claims from any claim being used as dependent claims for any other claim in the claims of this application.

Embodiments of the present invention will now be described herein, by way of example only, with reference to the following figures.

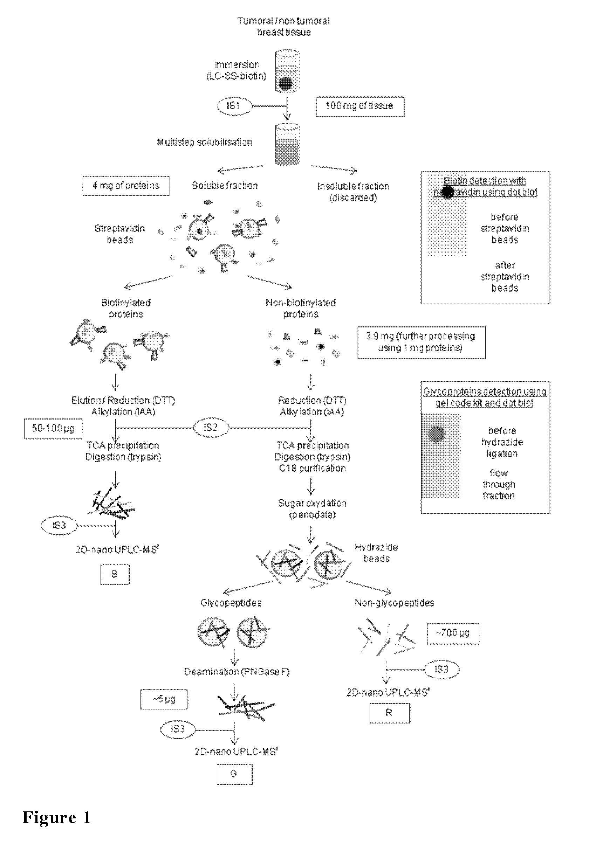

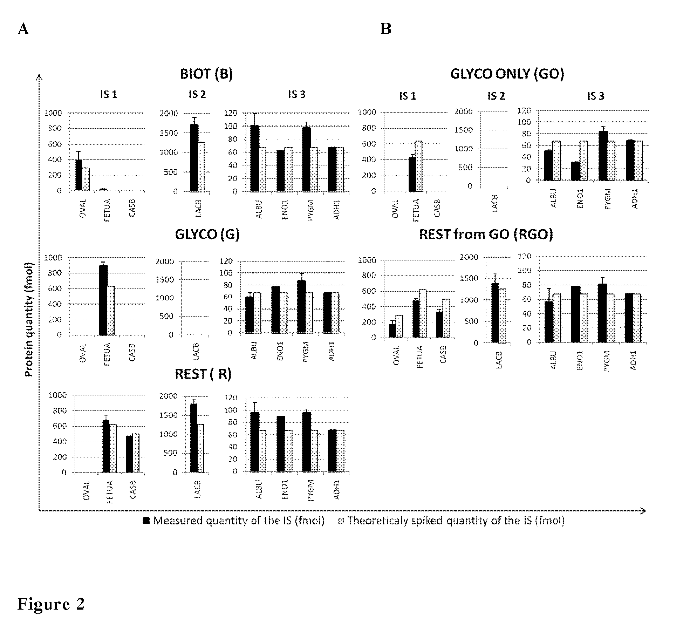

**Figure 1** - illustrates schematically the sequential extraction of accessible proteins from tissue samples. The first step consists of biotinylation of accessible proteins and their isolation (B fraction). The second step utilizes protein digestion of non biotinylated proteins into peptides, followed by the isolation of glycopeptides (G fraction). The final step also collects the non-glycosylated peptides (R) using them to complement the sequence information of non-assigned glycopeptides (detailed in Figure 2). Additionally, the R fraction contains a significant number of accessible proteins which supplement the already identified proteins in the B and G fractions. Three internal standards (IS1, 2 and 3) were added at different manipulation steps in the method in order to monitor, recovery, reproducibility and accuracy of quantification. The composition of the respective internal standard is outlined later. All the fractions were analyzed using the 2D-nanoUPLC-MS^{e} system, which consisted essentially of two C18-phases run at pH 10 and pH 3 respectively.
**Figures 2A and 2B** - illustrate the quantitative accuracy, recovery and reproducibility of the technique described in Figure 1. Figure 2A illustrates the absolute quantitative evaluation of the internal standards spiked in the sample during the preparation process using the sequential method (see Figure 1). Protein quantity was calculated using the PLGS software, based on a previously calculated response factor. The error indicates the standard deviation of means, based on three full process technical replicates. Figure 2B illustrates the absolute quantification of the internal standards spiked in the sample during the glycopeptides analysis alone. For comparative reasons the isolation of glycopeptides was also conducted as a standalone technique. Quantification and error bars are same as in Figure 2A.

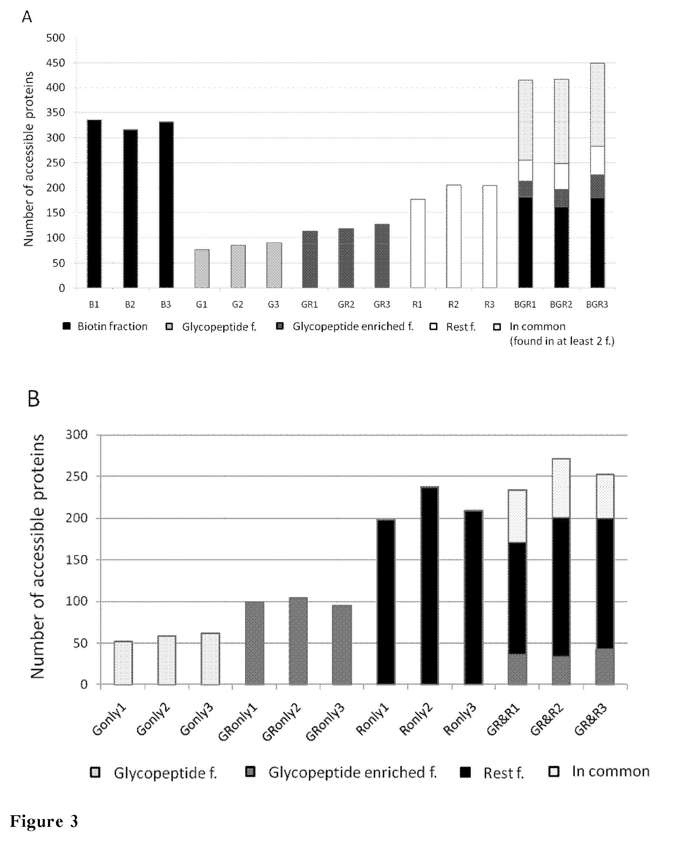

**Figures 3A and 3B** - illustrate the analysis of the effective value of the combinatory method of Figure 1A with respect to the individual components alone (biotin, glycopeptides and rest) and the absolute numbers of accessible proteins identified (accessible proteins: extracellular, secreted and/or membrane). **Figure 3A** illustrates the number of accessible proteins identified in each of the steps of the combinatory method as well as the sum of all the components together. Three full process technical replicates are displayed. Notably, G indicates the average number of proteins obtained using only the data from the trapped glycoprotein fraction. However, using the in-silico method, the overall number of glycoproteins was increased; this resulted in the data marked GR. Importantly, the specific dynamic range of proteins characterizing the R (rest) fraction allowed for a significant identification of additional accessible proteins. **Figure 3B** is the same as Figure 3A conducted only for the glycopeptides isolation procedure as standalone technique.

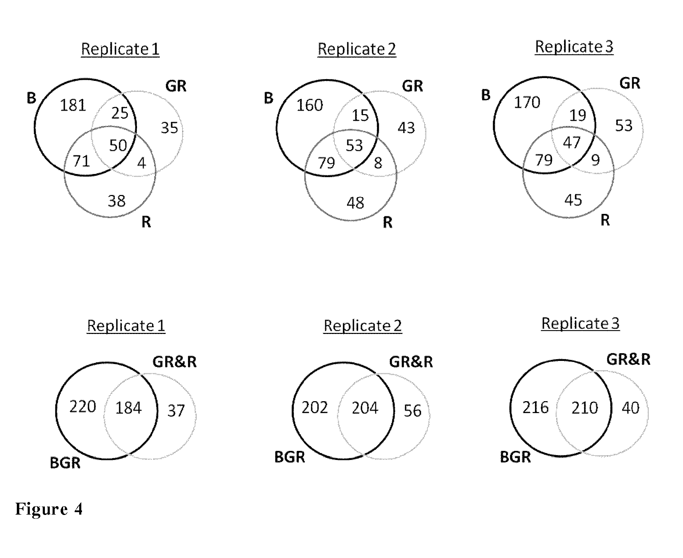

**Figure 4** illustrates the comparisons of the identification overlap of accessible proteins in different fractions of the combinatory method of Figure 1 and glycopeptides analysis alone. The Venn diagrams display three full process replicates. Regarding the combinatory method, Figure 4 shows that there is substantial overlap among all the separate fractions. However, each fraction also contains a significant number of unique proteins. Owing to the technical process the replicates demonstrate that there is significant added value in the individual steps of the combinatory method and that this is reproducible and beyond the measured variability of the analysis. The lower part of the figure shows a comparison between the combinatory method (BGR) and glycoprotein analysis alone (GR&R). The diagrams display only accessible proteins. For correct comparison accessible proteins found in the rest fractions are always included. It is worth noting that glycoprotein analysis as a standalone technique identifies on average 18 % of unique proteins. In contrast to this, over 50 % of the proteins identified in the combinatory method are unique.

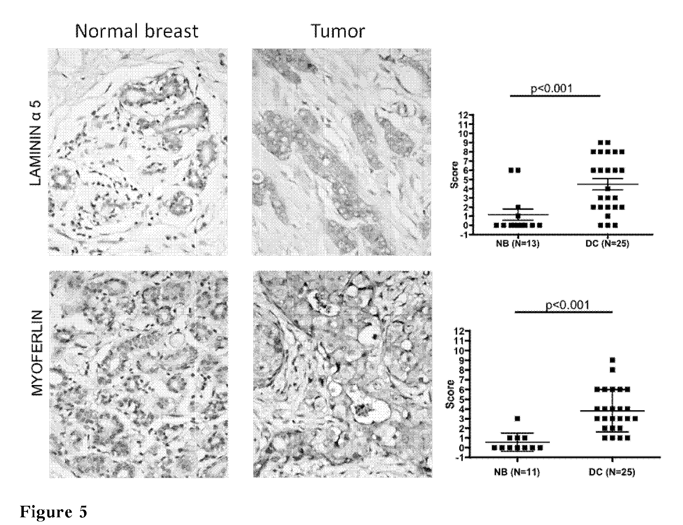

**Figure 5** - illustrates immunohistochemical validation that the proteins laminin α-5 and myoferlin are overexpressed in breast cancer tissue. Figure 5 shows representative cases from 25 tumoral and 11-13 normal individuals. Both laminin α-5 and myoferlin exhibit strong positive staining in the tumor tissue compared to the normal tissue (original magnification X400). The values in the graphs are reported as scores (NB= normal breast; DC= ductal carcinoma). The error bars indicate standard error of the mean. P-values ≤ 0.05 were considered as significant.

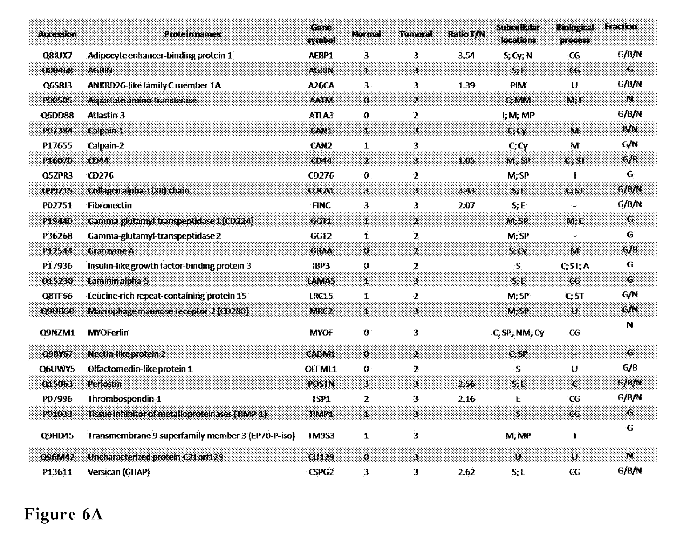

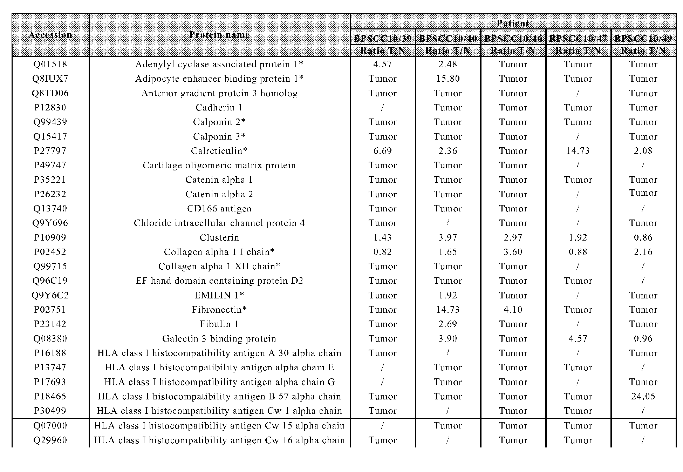

**Figure 6A** - is a list of potential breast tumor biomarkers obtained from the analysis of 3 non tumoral adjacent and 3 tumoral specimens. The selection of the potential biomarkers has been conducted with respect of the proteins presence in tumoral and absence or diminished presence in non-tumoral tissue samples. For certain proteins that have shown presence in both the tumoral and the adjacent normal tissue, semi-quantitative data (emPAI ratio) are included when these were significantly overexpressed in tumor tissue (ratio greater than 2.0). All the proteins are potentially accessible as they are located on the outer side of the cell membrane (secreted, extracellular or membrane).
   Following abbreviations were used:
   Subcellular location: S: secreted; E: extracellular; M: membrane; C: cell membrane; P1M: plasma membrane; SP: single pass membrane protein;
   MP: multipass membrane protein; LA: lipid anchor; MM: mitochondrion matrix; PM: peripheral membrane protein; Cy: cytoplasm; NM: nuclear membrane; U: cell component unknown.
   Biological process: C: cell communication, ST: signal transduction; I: immune response; M: metabolism; E: energy pathway; T: transport; A: apoptosis; CG: Cell growth and/or maintenance; U: biological process unknown.
   Fraction: G - glycopeptide; B - biotinylated; N - non glycopeptides;

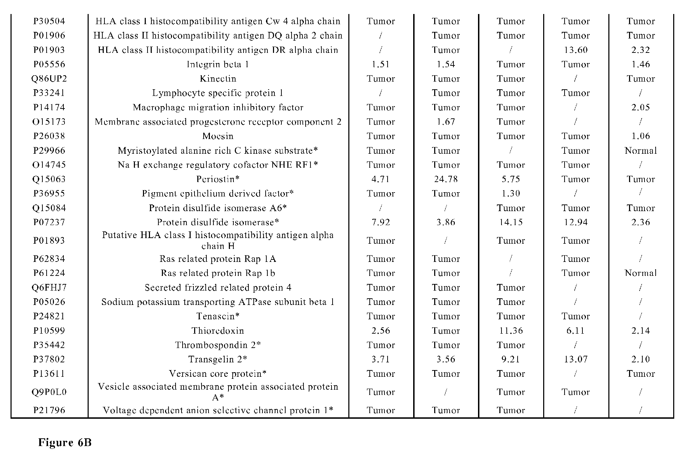

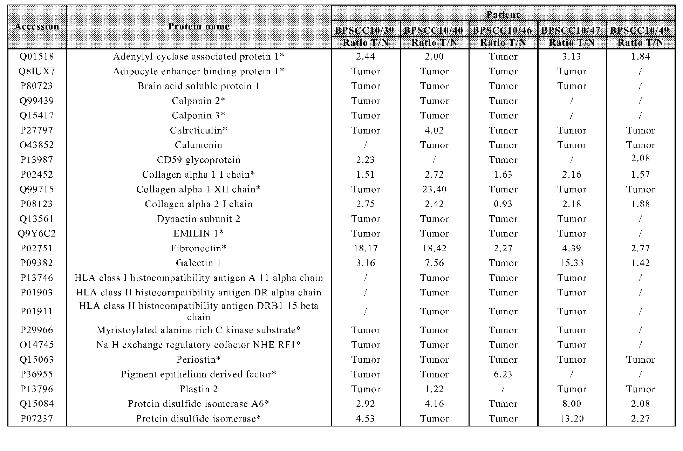

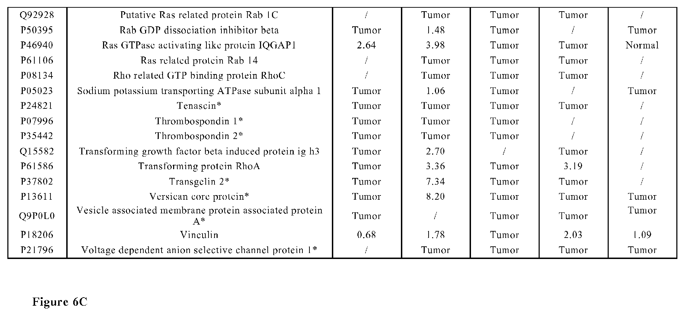

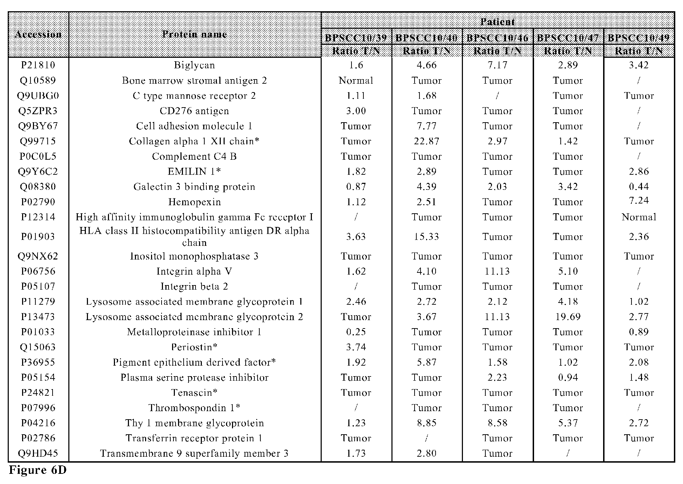

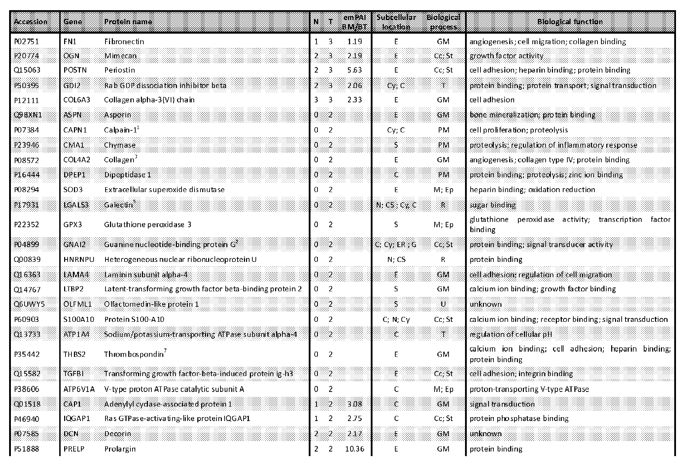

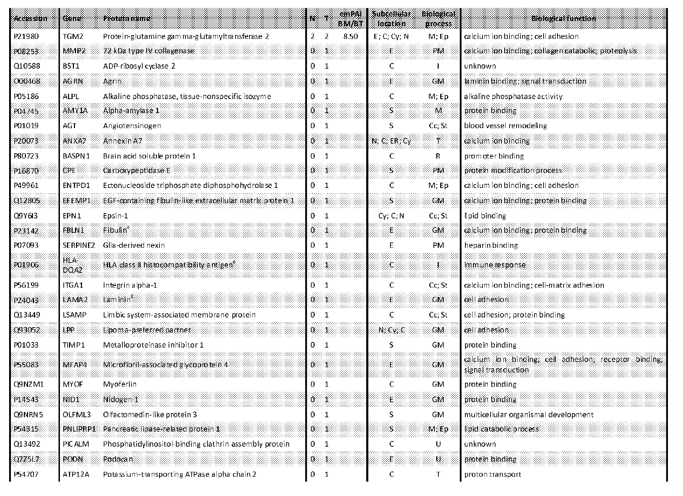

**Figures 6B-D** - list potentially accessible modulated proteins obtained from the analysis of 5 non-tumoral adjacent and 5 tumoral individual matched specimens; Figure 6B represents proteins isolated in the biotin fraction, Figure 6C represents proteins isolated in the rest fraction and Figure 6D represents proteins isolated in the glyco-fraction. The proteins were selected with respect to their presence in tumoral tissue samples and their absence or reduced presence in non-tumoral tissue samples. For certain proteins present in both the tumoral and the adjacent normal tissue, quantitative data (relative ratio of expression) are included if they were significantly over expressed in the tumor (ratio ≥ 1.5). All the proteins are accessible as they are located on the outer side of the cell membrane (secreted, extracellular or membrane). Following abbreviations were used: Subcellular location: S: secreted; E: extracellular; M: membrane; Cy: cytoplasm; CJ: cell junction; CP: cell projection; CSu: cell surface; N: nucleus; G: Golgi; ER: endoplasmic reticulum; Mel: melanosome; SR: sarcoplasmic reticulum; L: lysosome;

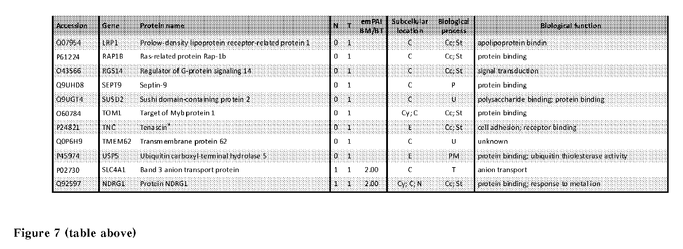

**Figure 7** - illustrates the accessible differentially expressed proteins identified as over expressed in pancreas tumor (n = 3) compared to normal tissue (n = 3). A protein is considered overexpressed when: i) it was detected only in the tumoral condition, ii) found in both conditions but more often identified in tumoral samples than normal samples, iii) found with a fold change ratio of the emPAI tumor/normal > 2. The following proteins were identified as multiple isoforms/subunits: ¹ Calpain-1 and Calpain-2 catalytic subunit; ² Guanine nucleotide-binding protein G (i) subunit alpha-2, (olf) subunit alpha, (k) subunit alpha, (o) subunit alpha, (q) subunit alpha, (t) subunit alpha-1and (t) subunit alpha-2; ³ Collagen alpha-2(IV) chain and alpha-1(XIV) chain; ⁴ Fibulin-1 and -2; ⁵ Galectin-3 and -4; ⁶ HLA class II histocompatibility antigen DQ alpha 2 chain and DR alpha chain;⁷ Thrombospondin-2 and -1; ⁸ Laminin subunit beta-2 and alpha-1; ⁹ Tenascin and isoform X.
   Potential subcellular location: C = Cell membrane. CS = Cell Surface. E = Extracellular. N = Nucleus. Cy = Cytoplasm. S = Secreted. ER = Endoplasmic Reticulum. G = Golgi; Biological process: U = Unknown. R = Regulation of nucleobase, nucleoside, nucleotide and nucleic acid metabolism. Cc; St = Cell communication, Signal transduction. GM = Cell growth and/or maintenance. P = Cell proliferation. I = Immune response. M; Ep = Metabolism, Energy pathways. Pm = Protein metabolism. T = Transport;

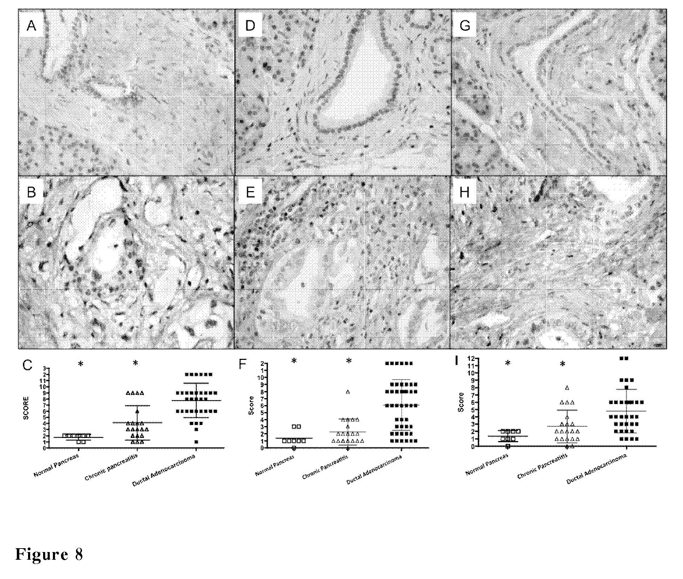

**Figure 8** - illustrates the immunohistochemical evaluation of the identified modulated proteins in pancreas non-cancerous tissue and adenocarcinoma. The image shows ASPN expression in normal peri-ductal tissue (A) and pancreatic adenocarcinoma (B). Comparison between the two groups resulted in a p-value < 0.0001 (C). TGFBi was predominantly negative in the normal tissue (D) but strongly present in tumoral tissue (E) with a p-value < 0.0001 (H). Immunostaining against LTBP-2 resulted in a significantly higher expression of the protein in tumoral (H) in comparison to the non-cancerous tissue (G). Comparison between the two groups resulted in a p-value = 0.0005 (I).

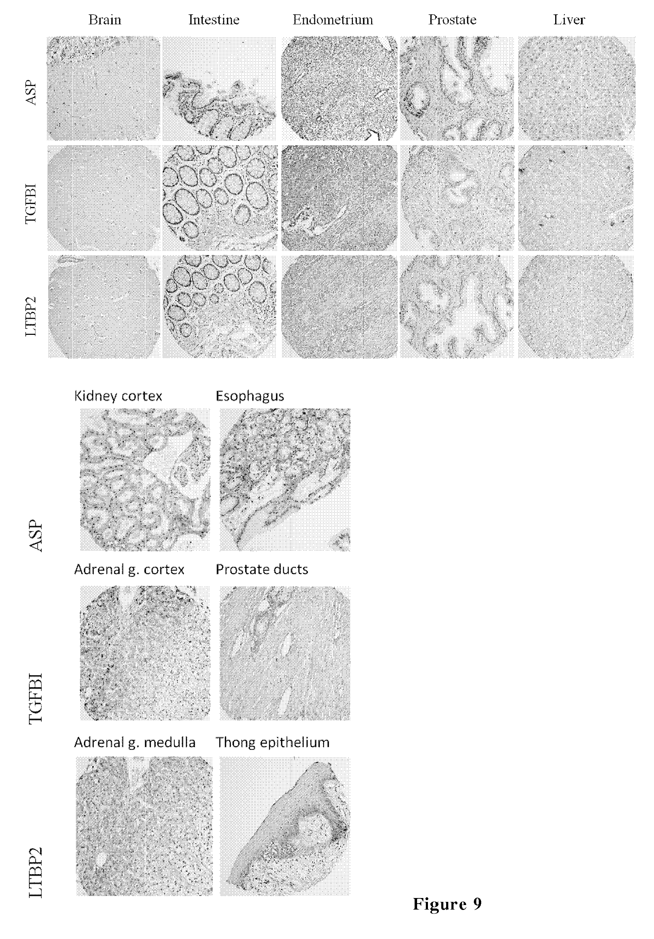

**Figure 9** - illustrates immunohistochemistry performed on the normal tissue TMA using antibodies against ASPN, TGFBI and LTBP2 targets. Here displayed are examples of the most important tissues scored. Brain, intestine, endometrium and prostate tissues were scored negative for all the antigens investigated. TGFBI expression was observed in liver, however was of weak intensity. Kidney cortex and oesophagus resulted in a weak expression of ASP (score 2 to 4). TGFBi expression was found in the cortex of adrenal gland and prostate ducts (score 4 to 6). A weak expression of LTBP2 was observed in medulla of the adrenal gland and thong epithelium (score2).

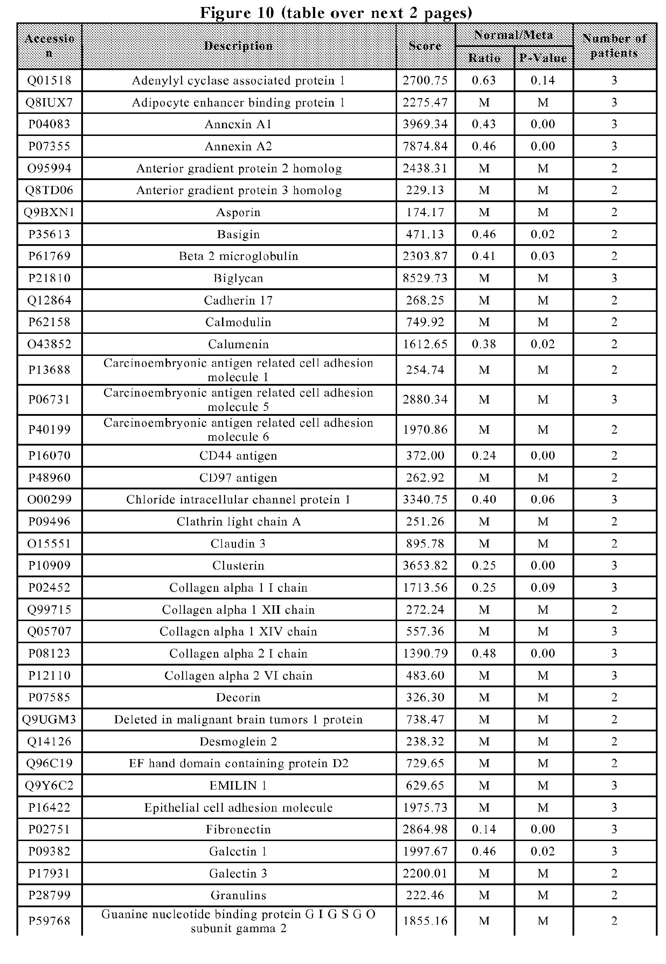

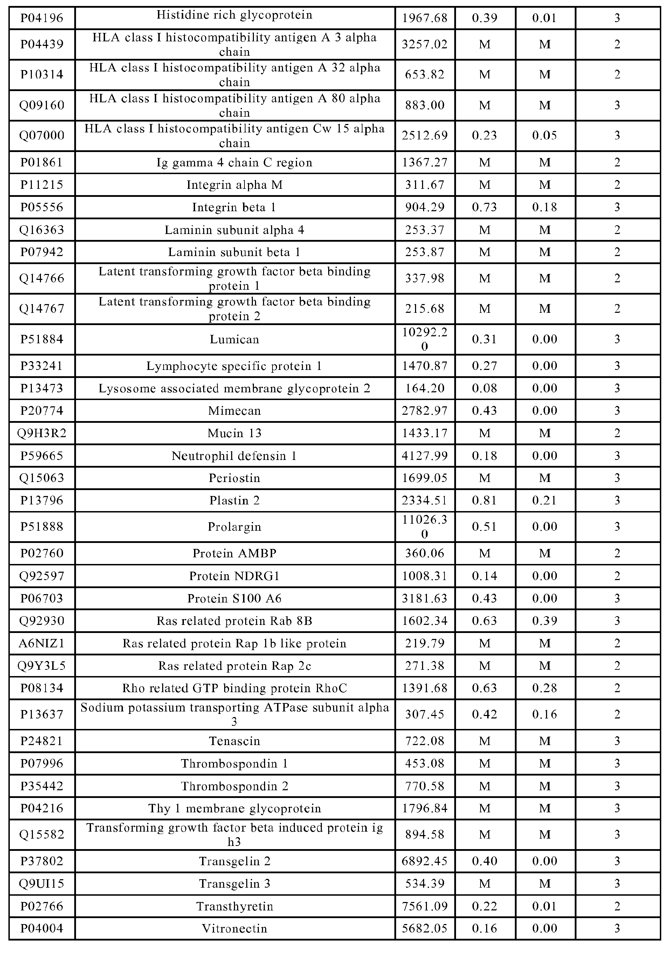

**Figure 10** - illustrates the accessible differentially expressed proteins identified in liver tumour (meta) tissue (n = 3) compared to normal tissue (n = 3).

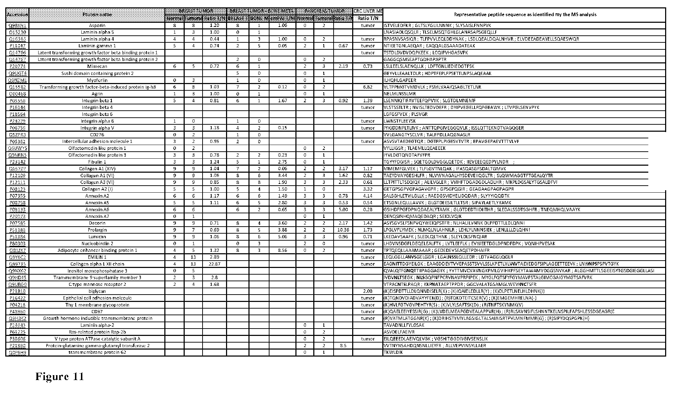

**Figure 11** - details accessible proteins obtained from the analysis of various different tumour tissues using the method of the invention, and details the peptide sequences found which led to the identification of the protein biomarker.

### BIOMARKERS FOR BREAST CANCER

The following study illustrates the invention, both the method of the invention and novel breast cancer biomarkers. The study isolated accessible protein biomarkers from breast cancer biopsy samples, and demonstrated their use as biomarkers for breast cancer.

All the individuals involved in the current work were informed in detail regarding the aims of the study and gave their written consent. The purpose of this project and the experiments undertaken complied with the regulations and ethical guidelines of the University of Liège, Belgium. The study is divided into two parts: i) technical (demonstrating the value, reproducibility and accuracy of the method) and ii) biological (proof of concept study for the discovery and validation of novel biomarkers). The technical part employed one "master sample" which was prepared as a pool of equal amounts of all the tissue samples involved in the MS analysis (all individuals, both tumoral and normal specimen). The biological part and specifically the discovery of modulated proteins was conducted using proteins isolated in the technical part. Finally, the validation of selected differentially expressed proteins was conducted on a separate group of breast cancer patients (25 tumoral and 13 normal individuals). All patients involved in the immunohistochemistry validation study were diagnosed with ductal breast adenocarcinoma, had clinical grades of at least 2 and presented no metastasis at the time of surgery.

Regarding the number and type of replicates involved in the study it is to be noted: i) all analyses conducted in the technical part involved respectively three full technical replicates (from tissue solubilization to MS analysis), performed on separate days and using the "master sample"; ii) the investigations conducted in the biological part, specifically discovery phase using MS, are single replicates of matched tumoral and normal samples originating from five individuals;

**Tissue sample preparation.** Pieces of fresh human breast cancer biopsies obtained from the Pathology Department of the University Hospital of Liège, Belgium, were immediately sliced and soaked in freshly prepared EZ-link Sulfo NHS-SS biotin (1 mg/ml, Pierce, Rockford, IL, USA) solution. Tissue samples were then snap-frozen in liquid nitrogen and pulverized using a Mikro-Dismembrator U (Braun Biotech, Melsungen, Germany). Approximately 100 mg of tissue powder was dissolved in the PBS buffer (50 mM PBS, 0.5 M NaCl, pH=7) containing protease inhibitor (PI) cocktail (Halt^{™}, Pierce, Rockford, IL, USA), 0.5 mM oxidized glutathione (GSSG) and level 1 internal standard mix (IS1 consisting of bovine alpha-2-HS-glycoprotein, bovine casein and biotinylated chicken ovalbumin [performed by incubation of ovalbumin with EZ-link Sulfo NHS-SS biotin reagent]; ratio spike/sample - 1/200). Homogenates were than sonicated (2 x 30 s) with a 2 mm microprobe and centrifuged at 20,000 x g for 10 minutes at 4°C. Human serum albumin (HSA) and immunoglobulins (IgGs) were eliminated using Qproteome HSA and IgGs Removal Kit (Qiagen, Valencia, CA, SA). The remaining pellet was suspended in the RIPA buffer (1% Nonidet P40 (NP40), 0.5% deoxycholic acid (DOC), 0.1% SDS, 0.5 mM GSSG and PI cocktail in PBS, pH=7.0), sonicated (2 x 30 s) and centrifuged at 20,000 x g for 10 minutes (at 4°C). The sample was subjected to HSA and IgGs depletion as described above. 2 % SDS solution was added to the remaining insoluble pellet following a final re-solubilization. The sample was then centrifuged (as mentioned above) and the supernatant collected. All lysates from the three solubilization steps were finally pooled together and boiled for 5 minutes.

**Isolation of biotinylated proteins.** The total protein extract was mixed with 100 µL/mg Streptavidin (SA) resin (Pierce, Rockford, IL, USA) for 120 minutes under rotational conditions at room temperature. After the streptavidin incubation, the supernatant was retained for the subsequent glycoproteomic analysis (fraction 1). The streptavidin beads were washed 4 times with 0.5 mL buffer A (1% NP40, 0.1% SDS and 0.5 mM GSSG in PBS buffer), 4 times with 0.5 mL buffer B (0.1% NP40, 1.5 M NaCl and 0.5 mM GSSG in PBS buffer), 2 times with 0.5 mL buffer C (0.1 M Na₂CO₃ and 0.5 mM GSSG in PBS buffer at pH=11.0) and finally 2 times with 0.5 mL PBS buffer at pH=7 without GSSG. The biotinylated proteins were eluted 2 times with 0.4 mL of 100 mM dithiothreitol (DTT) and incubated at 60°C for 30 minutes (fraction 2). Fraction 1 was also reduced in 100 mM DTT. Both fractions were alkylated with 150 mM iodoacetamide for 30 minutes in the absence of light. At this stage level 2 internal standard (IS2 consisting of bovine beta-lactoglobulin [1/200]) was added to both fractions. Proteins were then precipitated with 20% trichloroacetic acid (TCA) at 4°C overnight. The protein pellets (fractions 1 and 2) were solubilized in 50 mM NH₄HCO₃ and digested using trypsin (Promega, Madison, WI, USA) (1:50 protease/protein ratio) at 37°C overnight. The biotinylated peptides (fraction 1) were further processed using MS. Fraction 2 was used for the isolation of glycopeptides as described below.

**Isolation of glycopeptides.** The digested protein sample was acidified with 30 µL of 1 % HC1, transferred onto the C18 Sep-pak column (Waters, Milford, Massachusetts) and washed with 3 x 1 mL of 0.1 % formic acid solution. The peptides were eluted using acetonitrile (80%) and evaporated to dryness. The peptide-containing sample was dissolved in the oxidation buffer (100 mM sodium acetate, 150 mM NaCl at pH=5.5) and incubated with 10 mM sodium periodate (Pierce, Rockford, IL, USA) for 1 hour in the dark. Following this, 10 µL of 120 mM sodium sulfite was added and incubation was extended for an additional 10 min. The sample was loaded onto hydrazide resin (Bio-Rad, Hercules, CA, USA) and the glycopeptides were bound at RT overnight. The glycopeptide-free flow-through was collected for the subsequent MS analysis (non-glycosylated proteins, NGP or R). After extensive washing (2 times each with H₂O, 1.5 M NaCl, methanol, 80% ACN and 50 mM NH₄HCO₃) the hydrazide resin was loaded with 50 mM NH₄HCO₃ solution and incubated overnight with 500 units of PNGase F (New England Biolabs, Ipswich, MA, USA) at 37 °C. After the incubation period, the glycopeptide-containing flow-through (G) was collected and desiccated.

**MS analysis.** Peptides originating from biotinylated, glycosylated and also the non-glycopeptide fractions were desalted using C18 ZipTip pipette tips (Millipore, Billerica, MA, USA) prior to mass spectrometry analysis. Following this, 5 µg of peptide containing samples were dissolved in 18 µL of 100 mM ammonium formiate buffer. To the dissolved samples level 3 internal standard mix was added (IS3 composed of MassPREP Digestion Standard Mixture 1™ [Waters Corporation] - containing equimolar mix of yeast alcohol dehydrogenase, rabbit glycogen phosphorylase b, bovin serum albumin and yeast enolase; final concentration in 18 µL sample was adjusted to 135 fmol of yeast alcohol dehydrogenase). Of the sample prepared, 9 µL was injected corresponding to an estimated protein load of 2.5 µg. For the MS analysis the nano Aquity® UPLC (Waters) was coupled on-line with the SYNAPT G1 qTOF system (Waters). The configuration of the UPLC system was the following: trap column Symmetry® C18 5 µm, 180 µm × 20 mm (Waters), analytical column BEH® C18 1.7 µm, 75 µm × 150 mm (Waters), solvent A (0.1 % formic acid in water) and solvent B (0.1 % formic acid in ACN). The flow rate was set at 300 nL/min and the gradient had the following composition: 0 min, 97 % A; 90 min, 60 % A. The MS acquisition parameters were: data independent, alternate scanning (MS^{E}) mode, 50-1500 *m*/*z* range, ESI+, V optics, scan time 1 s, cone 30 V and lock mass [Glul]-Fibrinopeptide B [M+2H]²⁺ 785.8426 m/z. Raw data was processed (deconvoluted, deisotoped, protein identification, absolute and relative quantification) using ProteinLynx GlobalSERVER® (PLGS) v2.4. The processing parameters were: MS TOF resolution and the chromatographic peak width were set to automatic, low-/elevated- energy detection threshold to 250/100 counts, identification intensity threshold to 1500 counts and lock mass window to 785.8426 ± 0.35 Da. For protein identification UniProt® human database served as the reference (canonical sequence data with 20,280 enteries). Peptide modification carbamidomethylation was set as fixed and oxidation (M) as variable. In addition, for glycoprotein analysis, deamidation (N) was included as a variable modification as well. A response factor (2200) for the conversion of the peptide intensities into absolute quantities was deduced previously following a repeated injection of the alcohol dehydrogenase (yeast, SwissProt P00330) digest. This response factor was kept constant throughout the entire study. Routinely, IS1, IS2 and IS3 were checked for the correct relationship between the spiked and the measured absolute amount as well as for the relative ratio between the compared samples. The IS tolerances for both absolute quantities and relative ratios had to be within ± 25 % deviation in order for the data set to be acceptable and included in further analysis. PLGS® software calculated score and false positive rate (FPR) for each individual protein hit. Within the present study a protein was considered as identified if the FPR was ≥ 96 % and the score ≥ 80.

**Glycoprotein data analysis.** Regarding the glycoproteins, the processed MS data (deconvoluted spectra) were submitted for the database search, first separately for the fraction obtained from the hydrazide beads (G) and then combined with the flow-through fraction (R). Following this, all the glycoproteins originating from the hydrazide beads were filtered out with a home-made program (ARAC v3.5, available on request). This program checked for the presence of deamidated asparagines at the consensus sequence site (NXS/T, where X can be replaced by any amino acid except proline) for each of the peptides in question. In this initial step, a certain number of glycopeptides could immediately be assigned to a respective glycoprotein (G fraction). The remaining glycopeptides were not specific enough or had lower scores so that they could not be unambiguously associated with a protein. In order to help assign these peptides to a protein they were matched with the peptides from the R fraction analysis where several non-glycosylated peptides in conjunction with the glycosylated peptides (from the G fraction) permitted significant protein identification. This new combined pool of proteomic results was named the GR fraction.

**Immunohistochemical validation of selected biomarkers.** The expression of laminin α-5 and myoferlin was assessed by immunohistochemistry in formalin-fixed paraffin-embedded breast tissue sections. Samples originating from 25 tumoral and 13 normal breasts were immunostained using anti-laminin α-5 (Millipore, Billerica, US, dilution 1/100) and anti-myoferlin (Abcam, Cambridge, UK, dilution 1/400). Tissue sections of 5 µm thickness were unparaffined by two baths in xylene during 5 min and hydrated in the methanol gradient (100%, 95%, 70%, 50% and H₂O). Blocking of endogenous peroxidase was performed by 30 minutes incubation with 3% H₂O₂ and 90% methanol. Antigen retrieval was conducted in 10 mM citrate buffer (pH 6) using 95°C water bath for 40 min. Following 30 min blocking in PBS-normal serum solution (150µl normal goat serum and 20µl Tween 20 in 10ml PBS) the sections were incubated with the primary antibody overnight at 4°C. Sections were then incubated with the biotinylated secondary antibody for 30 minutes and further with avidin biotin complex kit (ABC kit) for additional 30 minutes. 3,3'-diaminobenzidine tetrachlorhydrate dihydrate (DAB) with 5% H₂O₂ was used for colorization. The slides were finally counter-stained with hematoxylin. Immunostaining was assessed by evaluating the samples for percentage of positive cells (five arbitrary units/ classes: 0 = 0%, 1 = 0-25%, 2 = 25-50%, 3 = 50-75% and 4 = 75-100%) and for staining intensity (four arbitrary units/ classes: 0 = no staining, 1 = weak, 2 = moderate and 3 = strong). The results obtained by these two scales were then multiplied together yielding a single value named score (y axis in the Figure 5). Statistical analyses were performed using unpaired two-tail Student t test for comparison between groups. Gaussian distribution was verified by the D'Agostino-Pearson test. Two-tail Mann-Whitney U test was used when Gaussian distribution and/or heteroscedasticity were not confirmed (in the case when the normal sample did not display any positive cells or/and no staining). P-values ≤ 0.05 were considered as significant. Statistical analyses were conducted using PRISM software (GraphPad Software, San Diego, California, version 4.0b).

### RESULTS

### Isolation of accessible proteins from tissue samples - the sequential method

The schematic overview of the sequential method is displayed in Figure 1. The method is composed essentially of three distinct steps: i) isolation of biotinylated proteins, ii) purification of the glycopeptides and iii) analysis of the remaining peptides. The latter fraction served for in-silico complementation of the non-assigned glycopeptides. In addition, as this fraction contained a significant number of accessible proteins, it was allowed to contribute this group of interest to the overall pool of modulated proteins. Due to the complexity of the method several rapid tools for monitoring the inter-replicate reproducibility were introduced. These consisted chiefly of: i) examining the flow-through of the biotin step for remaining biotinylated proteins and ii) measurement of the flow-through fraction after the hydrazide capture for residual glycopeptides. Further process controls were the internal standards which were spiked at three different steps, amounting to 8 individual proteins of non-human origin. Overall it can be said that the capture of both biotinylated proteins and glycopeptides was efficient and allowed for almost perfect specificity and minor samples loss. This is particularly obvious from the data presented in the Figure 2A. Here it is noteworthy that in the biotinylated protein fraction, quantitative recoveries of biotinylated albumin and negligible contamination of fetuin was detected (IS1). As far as the other two fractions are concerned (glyco and rest), fetuin was reproducibly quantified in both fractions (owing to both glycosylated and non-glycosylated peptides) whereas casein (IS1), being a pure phosphoprotein, was only recovered in the rest fraction. Regarding the reproducibility of the protein digestion and subsequent purification steps, good recoveries of beta-lactoglobulin (IS2) in all fractions except the glycopeptide one (for it is not a glycosylated protein), indicate that the quantitative and qualitative variability introduced by these steps is within the acceptable limits.

The present study used MS^{e} technology to perform absolute label-free quantification. The method used highly reproducible HPLC and data independent alternate scanning. In addition high mass accuracy measurements are provided by an orthogonal time-of-flight mass spectrometer and "on the flight" acquisition of lock-mass allowing for subsequent recalibration. The data was processed based on the detection and correlation of all detectable precursor and fragment ions sharing the same chromatographic profile. Rapid alteration between low- and elevated energy states applied to the collision cell allowed for the simultaneous quantification and identification of proteins in a single experiment. IS3 was observed to spike in the sample at the very late step of the sequential method (last step before the sample is injection in the UPLC) which allowed for a good estimate of the performance of the quantification approach. As outlined in the Figure 2A/B, the quantification of four proteins demonstrated that absolute quantification is feasible and accurate. However, a slight overestimation of the protein amounts especially for albumin (bovine) and glycogen phosphorylase b (rabbit) is evident. This could be related with the presence of human homologues found abundantly in the sample. Following the rationale that this variation is well below the 2-fold ratio chosen as the threshold for claiming differential expression of a protein, this deviation was not considered as significant.

Actual results using the method of the invention and the peptides found are given in Figure 11.

### Comparison of the sequential approach with the individual methods

In order to determine the exact benefit of combining two known procedures in a new method, the sequential technique was compared with the individual method parts respectively. For this purpose it was necessary to perform the second part of the method, the isolation of the glycopeptides, as a "standalone" technique. As far as the technical aspects of the method are concerned, the direct isolation of glycosylated peptides and the analysis of the remaining rest-fraction produced similar results as the sequential method. As shown in Figure 2A and 2B the glyco-fraction recovered specifically glycosylated fetuin, whereas biotinylated ovalbumin and casein were in-addition to fetuin only present in the rest-fraction. Internal standard 2 and 3 indicate reproducible digestion, purification and MS quantification.

As far as the absolute numbers of accessible proteins is concerned, the biotinylated protein fraction in the combinatory method isolated on average 310 proteins. The number of accessible proteins in the glycopeptide fraction (both in the sequential and standalone approach) was approximately 80 which increased to 110 following the in-silico combination method. This clearly demonstrates the value of performing this operation especially for non-assigned glycopeptides (an increase of ∼30%). In the rest fraction on average 200 potentially accessible proteins were confidently identified. The combination of all the method parts yielded in the sequential setting over 410 proteins (+ 30 % in comparison to the biotinylation alone) and in the glycopeptide setting alone (including the corresponding rest fraction) 210 proteins (- 50 % in comparison to the sequential method). The analytical procedure of removal of previously biotinylated proteins is clearly superior, in terms of the percentage of identified unique proteins (∼45 %), to both glycopeptide and the analysis of remaining (non-glycosylated and non-biotinylated) proteins. However, the joining of the glycopeptide isolation brings additional ∼36 % of unique, potentially accessible, proteins. The remaining pool of proteins (rest-fraction), due to a relatively high absolute numbers and high percentage of membrane and extracellular proteins, bears another ∼25 % of unique proteins of interest.

### Biomarker validation using immunohistochemistry

Using the MS-based semi-quantitative approach, laminin α-5 and myoferlin were found differentially overexpressed in the breast cancer samples. A study using 25 patients diagnosed with breast cancer, and the control group of 13 normal individuals, demonstrated that both laminin α-5 and myoferlin showed a strong positive staining in human breast cancer tissue. The corresponding normal breast tissue did not display any significant positivity for either of the antibodies used (Figure 5).

### BIOMARKERS FOR PANCREATIC CANCER

The method of the invention was also used to identify biomarkers for pancreatic cancer. The method used was as described above with reference to breast cancer except the samples were of pancreatic cancer tissue.

In this study, three normal pancreas tissues and three tumor lesions were analyzed in the proteomic discovery phase. A total of 736 and 841 proteins were identified in normal and tumoral conditions, respectively. Among these, more than 18% were found in all three normal (157 proteins) and tumoral (152 proteins) samples. Moreover, more than 29% of proteins of normal (239 proteins) and tumoral (244 proteins) samples were identified in two out of three biological replicates. This implied that 47% of the proteins found in each respective sample were also observed in at least another replicate. In total, 1139 unique proteins were identified. Their modulation was assessed by their "presence" or "absence" in tumoral with respect to normal condition. Moreover, emPAI values were used to semi quantify proteins that were found in both conditions. The emPAI value is an estimation of the protein abundance in complex mixtures. This value is calculated as a logarithm of the ratio of observed and theoretically observable peptides within the MS analysis. In the present study, a protein was considered overexpressed when it was (i) detected only in the tumoral condition, (ii) found in both conditions but more often indentified in tumoral samples, (iii) found with a fold change ratio of the emPAI (tumoral vs normal conditions) greater than two. Taking these criteria into account, 484 proteins were found as overexpressed in the pancreas tumor. Among these, 403 were exclusive to the tumoral samples while 81 were present in both conditions. The differentially expressed proteins were analyzed using Swissprot®/Uniprot® database in order to determine their potential subcellular localization. The potential accessibility of a given protein was evaluated according to the following criteria: the protein is localized in (i) cell membrane, (ii) on cell surface or it is described as (iii) secreted or (iv) extracellular. Secreted proteins known to be found abundantly in the serum were excluded from further analysis. Among the 484 proteins overexpressed in the tumoral condition, we were able to identify 84 proteins as potentially accessible whereas 310, 18 and 72 were described as unaccessible, serum and unknown proteins, respectively. The biological profiles of the 84 differentially expressed accessible proteins were determined according to their Gene Ontology annotation. The majority of the proteins were involved in cell communication and signal transduction (30%) as well as cell growth and/or maintenance (30%). The other biological processes were less represented: protein metabolism (9%), general metabolism and energy pathways (9%) and transport (6%).

Due to the unique method, the current study brought to light a considerable number of proteins (n=84) with potential accessible characteristics (Figure 7).

ASPN, TGFBI and LTBP-2 are novel biomarkers of pancreatic cancer, and their expression using immunohistochemistry in a series of tumoral (n=34) and non-tumoral (n=28) pancreatic tissues was studied (Figure 8). Furthermore the biodistribution of the antigen in question was assessed using normal tissue microarray. Staining intensity and extent was evaluated by scoring each slide. In the case of ASPN the immunoreactivity was exclusively associated to the extracellular matrix (ECM). Epithelial cells both tumoral and normal were negative. ASPN average expression was significantly stronger (p<0.0001) in tumoral in comparison with the non-cancerous tissue. However a low staining was detectable in normal ECM. Other normal tissue originating from adrenal gland, brain, endometrium, intestine, liver, placenta, prostate (both gland and ducts), pulmonary vein and artery, thymus, thyroid and umbilical cord tested negative for presence of ASPN (Figure 9).

As far as TGFBI is concerned the staining was generally found in the ECM as well as in the Langherans islands, which showed also a strong staining. Furthermore, in tumoral tissue epithelial cells showed low to moderate cytoplasmic staining. This was not observed in the normal tissue. A strong ECM expression was found in 50% of all the adenocarcinoma analyzed, while the remaining cases showed a rather low staining. Some ducts localized in the areas of chronic pancreatitis were moderately positive. Normal ducts showed no expression of the protein. Altogether, the average expression of TGFBI in tumoral tissue was significantly stronger (p<0.0001) when compared to the non-cancerous one. Regarding the expression of TFBI in other normal human tissue, the tissue microarray analysis indicated that colon, brain, breast, endometrium, myenteric plexus, myometrium, prostate (gland), thymus and thyroid were negative. A fairly modest positivity (score 2-4) was measured in adrenal gland, heart, liver, oesophagus, tongue and prostate (ducts).

LTBP-2 immunoreactivity was mainly detectable in ductal and peri-ductal stromal tissue. A low cytoplasmic staining was visible in few cases in normal gland and cancer cells. More than 50% of the adenocarcinoma showed a strong expression of the protein, which was significantly higher in comparison to the non-tumoral tissue (p=0.0005). However, low staining was visible in more than the half of the non-cancerous tissues. Concerning the expression of LTBP2 in normal tissue, the immunohistochemistry experiments revealed that this protein was not present in most of the tissues (adrenal gland (cortex), brain, breast, endometrium, heart, intestine, myenteric plexus, kidney, liver, placenta, prostate (gland), thymus, thyroid, umbilical cord and vein). Weak positivity (score ≤2) was registered in medulla of the adrenal gland, epithelium of the thong, mucosa of the bronchia and pulmonary artery.

### BIOMARKERS FOR CRC LIVER METASTASES

The method of the invention has also been used to identify biomarkers for liver metastases of colorectal carcinoma. The method used was as described above with reference to breast cancer except the samples were of liver cancer tissue. Figure 10 details of the accessible proteins identified using liver tissue.

## Claims

1. The use of one or more of myoferlin, CD276, macrophage mannose receptor 2 (CD280), transmembrane 9 superfamily member 3 (EP70-P-iso), EMILIN1, adipocyte enhancer-binding protein 1, agrin, , collagen alpha-1(XII) chain, laminin alpha-5, leucine-rich repeat-containing protein 15, , nectin-like protein 2, olfactomedin-like 1, inositol monophosphatase 3 and transforming growth factor beta induced protein ig-h3 (IHC) as a biomarker for breast cancer, or a predisposition thereto.

2. The use of claim 1 wherein one or more of myoferlin, agrin, laminin alpha-5, and olfactomedin-like 1 is a biomarker for breast cancer, or a predisposition thereto.

3. The use of myoferlin and/or laminin alpha-5 as a biomarker for breast cancer, or a predisposition thereto.

4. The use of claim 1 wherein adipocyte enhancer-binding protein is a therapeutic biomarker.

5. A method for determining the breast cancer status of a subject comprising the steps of:
(a) providing a sample of material from a subject;
(b) determining the level in the sample of one or more of the biomarkers of any of claims 1 to 3 and
(c) comparing the level determined in (b) with one or more reference values.

6. The method of claim 5 for use in any one or more of the following: to diagnose breast cancer in a subject; to assess the chance of a subject developing breast cancer; to advise on the prognosis for a subject with breast cancer; to monitor disease progression of breast cancer; and to monitor effectiveness or response of a subject to a treatment for breast cancer.

7. The method of claim 5 or 6 wherein the sample is a breast tissue biopsy.

8. The method of any of claims 5 to 7 wherein the reference value, to which the determined levels of the biomarker are compared, is the level of the same protein observed in one or more normal samples.

9. The method of any of claims 5 to 7 wherein the reference value is a previous value for a specific biomarker obtained for a specific subject.

10. The method of any of claims 5 to 9 wherein an increase in the level of the biomarker is indicative, or diagnostic, of breast cancer.

11. The method of any of claims 5 to 9 for use in monitoring breast cancer progression and/or to monitor the efficacy of a treatment administered to a subject.

12. The use of a ligand directed to a biomarker according to any of claims 1 to 4, for the manufacture of a medicament for the therapeutic and/or prophylactic treatment of a breast cancer.

13. A ligand directed to a biomarker according to any of claims 1 to 4, for the therapeutic and/or prophylactic treatment of a breast cancer.

14. The use of claim 12 or the ligand of claim 13 wherein the ligand is an antibody or antibody-drug conjugate.

15. A kit for use in determining the breast cancer status of a subject comprising at least one agent for determining the level of a biomarker according to any of claims 1 to 3 in a sample provided by the subject.
